# EUROPEAN PATENT SPECIFICATION

(11) **EP 2 072 014 B1**
(45) Date of publication and mention of the grant of the patent: **01.12.2010**
(21) Application number: 09001906.8
(22) Date of filing: 22.06.2007
(51) Int. Cl.: A61B 17/064

(54) **Novel skin staples**
Neue Hautklammern
Nouvelles agrafes pour peau

(30) Priority: 11.07.2006 US 819965 P
(43) Date of publication of application: 24.06.2009
(62) Divisional of application: 07252537.1
(73) Proprietor: Tyco Healthcare Group LP, North Haven, CT 06473 (US)
(72) Inventor: Prommersberger, Megan, Wallingford, CT 06492 (US)
(74) Representative: Alcock, David

(56) References cited:
- WO-A-2004/052594
- WO-A-2004/105621
- US-A- 4 719 917
- US-B1- 6 626 916

## Description

### TECHNICAL FIELD

The present disclosure provides a surgical skin staple. The staple may be constructed of a composite including a metal and a bioabsorbable shape memory polymer. Methods of stapling tissue with a staple of the present disclosure are also provided.

### BACKGROUND

Surgical staples are used to close wounds, both internally and in the skin. Some staples are metallic, including, for example, the staples disclosed in U.S. Patent No. 4,321,002. Other staples may be made of polymeric materials, including nonabsorbable materials or the bioabsorbable polymers of U.S. Patent No. 6,235,689. Staples constructed of both nonabsorbable and absorbable materials are also known; see, for example, U.S. Patent No. 4,719,917 which is considered to represent the closest prior art. Staples may be utilized in conjunction with other closure methods, including sutures and adhesives, or the staples may be utilized by themselves to close wounds in tissue, including skin.

In order to close, staples used for stapling skin may only be acted upon by forces from one side of the stapled medium, whereas other types of staples may be acted upon by forces both above and below the stapled medium. Thus, one difficulty in the use of skin staples is the ability of the staple to sufficiently grab the tissue to which it is applied, as well as the ability of the staple to remain affixed thereto during healing. Staplers having excellent penetration characteristics and the ability to remain affixed to the tissue to which they are applied during healing, especially in the case of skin staples, remain desirable.

### SUMMARY

The present invention relates to a method comprising:
providing a surgical staple comprising:
   a backspan (4,6) comprising a non-absorbable material and two legs, at least a portion of each of the two legs comprising a bioabsorbable thermal shape memory polymeric material, wherein the said portions have a permanent bent or curved shape and are deformable to a temporary shape at a deforming temperature above the transition temperature (Ttrans) but below the permanent temperature (Tperm) of the thermal shape memory polymeric material; and
straightening the said portions to a temporary insertion shape at a deforming temperature above the transition temperature but below the permanent temperature of the thermal shape memory polymeric material.
.

Suitable non-absorbable materials which maybe utilized to form the backspan include metals such as titanium, stainless steel, steel alloys, titanium alloys, nickel chromium alloy, nickel/cobalt/chrome, and combinations thereof.

In embodiments, suitable shape memory polymers which may be utilized include copolymers of a diol ester and an ether-ester diol. In some embodiments, the shape memory polymer may include oligo (epsilon caprolactone) diol/oligo (p-dioxanone) diol copolymers, lactide, glycolide, and combinations thereof. Other suitable shape memory polymers include a blend of materials such as urethanes, lactic acid, glycolic acid, acrylates, caprolactones, homopolymers thereof, copolymers thereof, and combinations thereof.

### BRIEF DESCRIPTION OF THE DRAWINGS

FIG. 1 is a depiction of a staple of the present disclosure having a linear backspan and bioabsorbable shape memory polymeric legs attached thereto;

FIG. 2 is a depiction of a staple of the present disclosure having a backspan which is curved at its ends thereby forming the upper portion of the legs of the staple, with bioabsorbable shape memory polymeric materials forming the remainder of the legs of the staple;

FIG. 3 is a depiction of another staple of the present disclosure having a linear backspan and bioabsorbable shape memory polymeric legs attached thereto;

FIG. 4 is a depiction of a staple of the present disclosure in its memorized shape; and

FIG. 5 is a depiction of a staple of the present disclosure in-its temporary shape.

### EMBODIMENTS

In accordance with the present disclosure, staples are provided having an excellent ability to remain affixed to tissue for the duration of healing. The staples of the present disclosure include skin staples. The staples remain affixed to the tissue to which they are applied for an adequate period of time to permit healing. Being partly constructed of bioabsorbable materials, difficulties in the removal of the present staples (including the possibility of damaging tissue on removal), are avoided.

Briefly, the present disclosure provides-a novel composite staple including a non-absorbale portion and a portion formed from a shape memory polymeric material. The backspan of the staple, which may also be referred to herein as the crown, may be made of any biocompatible non-absorbable rigid or semi-rigid material, in embodiments a metal, while the legs are made at least in part of a bioabsorbable shape memory material. In use, the legs of a staple of the present disclosure may be temporarily deformed to make insertion into tissue easier. A memorized bent or curved shape of the legs may then be recovered after insertion to enhance the ability of the staple to grab tissue. As the legs are at least partially bioabsorbable, the non-absorbable backspan may be removed or-may fall off after sufficient hearing without any further damage to the tissue.

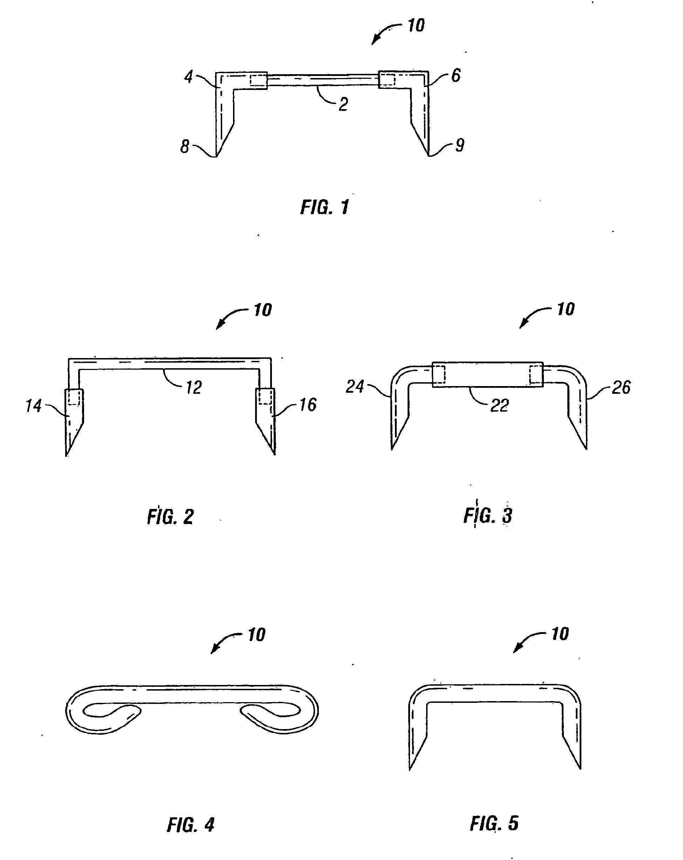

Referring now to the drawings, FIG. 1 shows a staple 10 of the present disclosure. Central backspan portion 2 is linear and is attached to shape memory bioabsorbable legs 4 and 6 having tips 8 and 9. Once deformed to their temporary straight shape, the legs attached to the backspan may project perpendicularly from the backspan forming a U-shape.

FIG. 2 depicts another configuration of a staple 10 of the present disclosure, wherein central backspan portion 12 is curved at its ends, with the curved portions forming the upper sections of the legs 14 and 16 of the staple. The backspan portion 12 may be a non-absorbable material such as a metal of solid or hollow stock and the remainder of legs 14 and 16 may be made of a bioabsorbable shape memory material.

FIG. 3 shows another staple 10 of the present disclosure. CentraLbackspan portion 22 is linear and-is attached to shape memory bioabsorbable legs 24 and 26. While staple 10 in FIG. 1 has central backspan portion 2 embedded in shape memory bioabsorbable legs 4 and 6, the staple 1 of FIG. 3 has shape memory -bioabsorbable legs 24 and 26 embedded in central backspan portion 22.

Suitable metals which may-be utilized to form the backspan of the staple include, for example, titanium, stainless steel, steel alloys, titanium alloys, nickel chromium alloy, nickel/cobalt/chrome, and combinations thereof, although other metals, plastics, or ceramics can be used. Titanium such as commercially pure titanium, sometimes referred to herein as CP titanium, may be utilized. CP titanium includes unalloyed titanium and may designate several grades of titanium possessing minor amounts of impurities; such as carbon, iron, and oxygen. The microstructure of CP titanium may be primarily a hexagonal-close-packed crystal structure with a relatively low strength and high ductility. The amount of oxygen may be controlled to provide increased strength. The general chemical composition (wt%) and minimum mechanical properties of the grades of CP titanium are set forth below in Table 1.

**TABLE 1**

| | Grade | | | |
|---|---|---|---|---|
| | 1 | 2 | 3 | 4 |
| Nitrogen, max. | 0.03 | 0.03 | 0.05 | 0.05 |
| Carbon, max. | 0.10 | 0.10 | 0.10 | 0.10 |
| Hydrogen, max. | 0.01 | 0.01 | 0.01 | 0.01 |
| Iron, max. | 0.20 | 0.30 | 0.30 | 0.50 |
| Oxygen, max. | 0.18 | 0.25 | 0.35 | 0.40 |
| Titanium | bal. | bal. | bal. | bal. |
| Yield strength (MPa) | 170 | 275 | 380 | 485 |
| Ultimate strength (MPa) | 240 | 345 | 450 | 550 |
| Elongation (%) | 24 | 20 | 18 | 15 |

The backspan may be linear or curved at its ends, thereby forming upper portions of the legs of a staple of the present disclosure, with the shape memory polymeric materials forming the lower legs of the staple of the present disclosure.

The legs of the staple are formed at least in part of bioabsorbable shape memory polymeric materials which may be resorbed in the body. Such materials include, for example, caprolactones, dioxanones, diol esters including oligo (epsilon caprolactone) diol, lactic acid, lactide, glycolic acid, glycolide, ether-ester diols including oligo (p-dioxanone) diol, carbonates including trimethylene carbonate, combinations thereof, and the like. The bioabsorbable shape memory polymer may be a copolymer of two components with different thermal characteristics, such as a diol ester including oligo (epsilon-caprolactone diol) and an ether-ester diol such as a crystallizable oligo (p-dioxanone) diol. These multi-block oligo (epsilon-caprolactone) diol/oligo (p-dioxanone) diol copolymers possess two block segments: a "hard" segment and a "switching" segment linked together in linear chains. Such materials are disclosed, for example, in Lendlein, "Shape Memory-Polymers-Biodegradable Sutures." Materials World, Vol. 10, no. 7, pp. 29-30 (July 2002). Alternatively blends of bioabsorbable materials may be utilized including, but not limited to, urethanes blended with lactic acid and/or glycolic acid, homopolymers thereof or copolymers thereof, and acrylates blended with caprolactones such as polycaprolactone dimethacrylate poly(butyl acrylate) blends, and combinations thereof.

The shape memory polymeric materials of the present disclosure may, in embodiments, be stretched or compressed into temporary forms up to about four times larger or four times smaller than their permanent shape. The shape memory polymeric materials are fashioned into legs of a staple of the present, disclosure having a permanent shape that is curved or bent, and a temporary shape that is straight.

A molding process may be utilized to produce legs with the permanent shape to be memorized, for example, a bent or curved shape for holding tissue. Plastic molding methods are within the purview of those skilled in the art and include, but are not limited to, melt molding, solution molding and the like. Injection molding, extrusion molding, compression molding and other methods can also be used as the melt molding technique. Once placed in the mold with the proper bent configuration, the legs may be heated to a suitable temperature, in embodiments at a temperature referred to as the permanent temperature (Tₚₑᵣₘ), for example, from about 40° C to about 60° C, in embodiments from about 45° C to-about 55° C, for a period of time from about thirty seconds to about sixty minutes, in embodiments from about 1 minute to about 10 minutes, to obtain their permanent curved or bent shape.

After the molded legs with the desired permanent shape are formed, the legs may be deformed at a deforming temperature to obtain a temporary shape. Suitable temperatures for deformation may vary depending on the shape memory polymer utilized, but generally may be above the transition temperature of the polymer (Tₜᵣₐₙₛ), but below the Tₚₑᵣₘ In embodiments, the shape memory polymer may be cooled from its Tₚₑᵣₘ to a lower temperature which remains above the Tₜᵣₐₙₛ and deformed to the temporary shape. In embodiments, the legs may be straightened and cooled to room temperature (about 20° C to about 25° C) to obtain their temporary straight shape, although the temperature may differ depending upon the particular polymer employed. The legs are then cooled to a temperature below Tₜᵣₐₙₛ, at which time the staple of the present disclosure may be ready for use.

The legs thus prepared recover their originally memorized bent or curved shapes on heating, either by placement in a patient's body or the addition of exogenous heat at a prescribed temperature above Tₜᵣₐₙₛ for the shape memory polymer utilized.

In other embodiments, the legs may be deformed to a straight shape and heated to assist in their adoption of their temporary shape. The temperature for deformation treatment of legs molded with a previously memorized shape is one that makes possible ready deformation without producing cracks and should not exceed the temperature adopted for the shape memorization. Deformation treatment at a temperature-exceeding that for the original shape memorization may cause the object to memorize a new deformed shape. Deformation by heating can be accomplished either by means of heaters of a special design or under an atmosphere of a suitable hot gas or liquid.

In other embodiments, legs utilized as part of a staple of the present disclosure may be formed by cold drawing, i.e., drawing a sample at a low temperature. Cold drawing methods are within the purview of those skilled in the art. Examples of these shape memory polymers and means for forming permanent and temporary shapes therewith are set forth in Lendlein et al., "Shape memory polymers as stimuli-sensitive implant materials," Clinical Hemorheology and Microcirculation, 32 (2005) 105-116, and Lendlein et al., "Biodegradable, Elastic Shape-Memory Polymers for Potential Biomedical Applications," Science, Vol. 269 (2002) 1673-1676.

There are no particular limitations on the manner in which the deformation can be achieved. Deformation can be achieved either by hand or by means of a suitable device selected according to the shape, size, thickness and other desirable characteristics of the molded legs.

In order to keep the shapes of the legs fixed in their temporary shape, the shape-memory molded legs of this invention should be stored at a temperature which will not cause plastic deformation of the polymers. The shape-molded memory legs may be stored in a refrigerator.

As staples of the present disclosure are utilized in a living-body, heating with body heat is possible. In such a case, the temperature for shape memorization should be as low as possible and the recovery of the memorized shape may occur fairly slowly.

However, a higher shape-memory temperature may be desirable in order to make the shape recover at a slightly higher temperature than body temperature. Thus, in some cases releasing the molded legs from deformation to recover the originally-memorized bent or curved shape can be achieved by heating. On heating at a temperature from about 30° C to about 50° C, for instance from about 39° C to about 43° C, the temporary shape may be released and the memorized permanent shape recovered. The higher the temperature for heating, the shorter the time for recovery of the originally memorized shape. The means for this heating is not limited. Like the heating for deformation treatment, heating can be accomplished by using a gas or liquid heating medium, heating devices, ultrasonic waves, or the like. Of course, in an application involving a living body, care should be taken to utilize a heating temperature which will not cause burns. When a liquid heating medium is used, physiological saline solution or alcohol may be desirable.

The leg portions of the staple flank the central backspan portion and possess points at their ends. In embodiments, the legs of the skin staple of the present disclosure may possess sharp, penetrating tips which degrade after implantation to an extent whereby the backspan may be painlessly removed. The degradation time of the legs can be varied, for example, by the selection of the shape memory material utilized to form the leg, the coupling means and configuration utilized to attach the legs to the backspan, and the like. Times for absorption can range from about 5 days to about 2 years, for instance from about 1 week to about 3 months for skin staples, depending on the composition of the leg portions of the staples and their shapes.

The backspan of the staple may be physically or chemically coupled to the legs of the staple utilizing methods within the purview of one skilled in the art.

Physical coupling of the backspan portion and the leg portion can be, for example, by use of a socket in the backspan portion that frictionally or telescopically receives the leg portion, or it can be a tongue and groove frictional coupling means, or any combination of any of these coupling means. Additional fastening means may be utilized to mechanically attach the backspan portion to the legs. Other locking mechanisms include a locking taper, a screw, a rivet, etc. Combinations of the above methods may be used to physically connect the backspan to the legs. For example, the coupling means can be a mechanical fastener (e.g., tongue and groove) in combination with, for example, a bioabsorbable pin or rivet.

Chemical means for joining the backspan to the legs may include, for example, injection molding the shape, memory legs onto the ends of the backspan portion of the staple. In an injection molding process, the leg portion of a staple may be adhesively coupled to the backspan portion by pressing the end of the backspan into an end of the leg portion opposite the pointed tip while the leg is in a molten state. The bond between the leg portions and the backspan portion can be an adhesive one that results from the cooling of the polymer in contact with metal. Part of the adhesion may result from shrinkage of the polymer upon cooling when it is in a configuration so as to surround the metal backspan portion. Other conventional methods such as casting from a solvent or melt pressing can also be used to join the leg to the backspan. Alternatively, shape memory polymeric legs may be inserted into hollow metal tubes at the ends of the backspan; this may be especially useful where the backspan portion is curved at its ends to permit placement of the shape memory leg into the upper leg portion found on.the backspan. In such a case, a bioabsorbable adhesive can be useful for adhering the shape memory polymeric legs to the backspan portion.

The legs may possess their permanent shape prior to their attachment to the backspan or the legs may be formed so that they possess their permanent shape as they are attached to the backspan or, in other embodiments, the legs may be created to possess their permanent shape after their attachment to the backspan. The legs are deformed to their temporary shape after their attachment to the backspan.

The implantation of a staple of the present disclosure in its temporary shape, and the transition thereof to its permanent shape in vivo, will now be described. The staple can be-dispensed from a conventional mechanical device into the skin to close a break in the skin such as a wound, i.e., the staple may be dispensed into the skin adjacent a wound and become affixed to the tissue, thereby closing the wound. Staples of the present disclosure having the shape memory legs described herein may have a temporary shape prior to implantation wherein the legs are straight; thus a staple 10 having such legs may possess a standard U shape configuration as depicted in FIG. 4.

Upon implantation in the body, the heat of the body (about 37° C) may result in the staple 10 with legs having the straight, temporary shape depicted in FIG. 4 to return to its permanent shape having legs with a bent configuration that will permit the staple to grab and adhere to tissue, thereby retaining the staple in tissue and closing the wound to which they are applied. A staple 10 of the present disclosure having legs formed of a shape memory polymer as described herein that has returned to its permanent bent of curved shape may have a closed configuration as depicted in FIG. 3 In other embodiments, heat may be applied to the staples, for example from about 39° C to about 43° C (just above human body temperature), to enhance the return of the shape memory polymer to its permanent bent or curved shape so that the staple 10 has a closed configuration as depicted in FIG. 3.

Again referring to FIG. 2, after sufficient healing has taken place, backspan 12 will have separated from the bioabsorbable shape memory legs 14 and 16 of staple 10 due to the degradation of legs 14 and 16 in vivo. Thus, backspan 12 may be removed simply by application of gentle traction on the exposed backspan portion 12 by, for example, plucking the metal using fingernails or tweezers, or by the action of a wash cloth used during bathing. An alternative method for rapid staple removal is to place a strip of adhesive tape on top of a row of staples utilized to close a wound in the skin and then to quickly strip off the tape which will remove all the backspan portions 12 of the staples.

The bioabsorbable shape memory legs 14 and 16, which are not necessarily completely absorbed at this time, remain buried under the skin and are slowly absorbed by the body. As noted above, times for absorption can range from about 5 days to about 2 years, in embodiments from about 1 week to about 3 months, depending on the composition of the leg portions and their shapes. Any shape memory polymer utilized to form-legs 14 and 16 that remains implanted should have no effect on the rapid healing of any holes made by the staple 10 upon attachment to tissue.

A variety of optional ingredients including medicinal agents may-be-combined with the shape memory polymers utilized to form the legs of staples of the present disclosure. The term "medicinal agent", as used herein, is used in its broadest sense and includes any substance or mixture of substances that have clinical use. Consequently, medicinal agents may or may not have pharmacological activity per se, e.g., a dye. Alternatively a medicinal agent could be any agent which provides a therapeutic or prophylactic effect, a compound that affects or participates in tissue growth, cell growth, and/or cell differentiation, a compound that may be able to invoke a biological action such as an immune response, or a compound that could play any other role in one or more biological processes.

Examples of classes of medicinal agents which may be utilized in accordance with the present disclosure include antimicrobials; analgesics; antipyretics; anesthetics; antiepileptics; antihistamines; anti-inflammatory agents such as hormonal agents, hydrocortisone, prednisolone, prednisone, non-hormonal agents, allopurinol, indomethacin, phenylbutazone and the like, cardiovascular agents such as coronary vasodilators and nitroglycerin; diagnostic agents; sympathomimetics; cholinomimetics; antimuscarinics; antispasmodics; hemostats to halt or prevent bleeding; muscle relaxants; adrenergic neuron blockers; antineoplastics; immunogenic agents; immunosuppressants; gastrointestinal drugs; diuretics; steroids; lipids; lipopolysaccharides; polysaccharides; and enzymes. It is also intended that combinations of medicinal agents may be used.

Other medicinal agents which may be included with the shape memory polymers utilized to form the legs of staples of the present disclosure include local anesthetics; non steroidal antifertility agents; parasympathomimetic agents; psychotherapeutic agents; tranquilizers; sedative hypnotics; sulfonamides; vaccines; vitamins; antimalarials; anti-migraine agents; anti-parkinson agents-such as L-dopa; antispasmodics; anticholinergic agents (e.g. oxybutynin); bronchodilators; alkaloids; narcotics such as codeine, dihydrocodeinone, meperidine, morphine and the like; non-narcotics such as salicylates, aspirin, acetaminophen, d-propoxyphene and the like; opioid receptor antagonists, such as naltrexone and naloxone; anticoagulants; anticonvulsants; antidepressants; anti-emetics; prostaglandins and cytotoxic drugs; estrogens; antibiotics; anti-fungals; anti-virals; and immunological agents.

Suitable antimicrobial agents which may be included as a medicinal agent with the shape memory polymers utilized to form the legs of staples-of the present disclosure include triclosan, also known as 2,4,4'-trichloro-2'-hydroxydiphenyl ether, chlorhexidine and its salts, including chlorhexidine acetate, chlorhexidine gluconate, chlorhexidine hydrochloride, and chlorhexidine-sulfate, silver and its salts, including silver acetate, silver benzoate; silver carbonate, silver citrate, silver iodate, silver iodide, silver lactate, silver laurate, silver nitrate, silver oxide, silver palmitate, silver protein, and silver sulfadiazine, polymyxin, tetracycline, aminoglycosides, such as tobramycin and gentamicin, rifampicin, bacitracin, neomycin, chloramphenicol, miconazole, quinolones such as oxolinic acid, norfloxacin, nalidixic acid, pefloxacin, enoxacin and ciprofloxacin, penicillins such as oxacillin and pipracil, nonoxynol 9, fusidic acid, cephalosporins, and combinations thereof. In addition, antimicrobial proteins and peptides such as bovine lactoferrin and lactoferricin B may be included as a medicinal agent with the shape memory polymers utilized to form the legs of staples of the present disclosure.

Examples of hemostat materials-which can be employed include fibrin-based, collagen-based oxidized regenerated cellulose-based, and gelatin-based topical hemostats. Examples of commercially available hemostat materials include fibrinogen-thrombin combination materials sold under the trade designations COSTASIS^{™} by Tyco Healthcare Group, LP, and TISSEEL^{™} sold by Baxter International, Inc. Hemostats herein also include astringents, for example, aluminum sulfate, and coagulants.

Other examples of suitable medicinal agents which may be included with the shape memory polymers utilized to form the legs of staples of the present disclosure include viruses and cells, peptides, polypeptides and proteins, analogs, muteins, and -active fragments thereof, such as immunoglobulins, antibodies, cytokines (e.g. lymphokines, monokines, chemokines), blood clotting factors, hemopoietic factors, interleukins (IL-2, IL-3. IL-4, IL-6), interferons (β-IFN, (α-IFN and γ-IFN), erythropoietin, nucleases, tumor necrosis factor, colony stimulating factors (e.g., GCSF, GM-CSF, MCSF), insulin, anti-cancer and/or anti-tumor agents and tumor suppressors, -blood proteins, gonadotropins (e:g., FSH, LH, CG, etc.), hormones and hormone analogs (e.g., growth hormone), vaccines (e.g., tumoral, bacterial and viral antigens); somatostatin; antigens; blood coagulation factors; growth factors (e.g., nerve growth factor, insulin-like growth factor); protein inhibitors, protein antagonists, and protein agonists; nucleic acids, such as antisense molecules, DNA and RNA; oligonucleotides; and ribozymes.

A single medicinal agent may be utilized with the-shape memory polymers utilized to form the legs of staples of the present disclosure or any combination of medicinal agents may be utilized with the shape memory polymers utilized to form the legs of staples of the present disclosure.

The medicinal agent may be disposed on a surface of a leg of a staple of the present disclosure or impregnated in or combined with the shape memory polymers utilized to form the legs of staples of the present disclosure.

Additionally, an enzyme may be added to the shape memory polymers utilized to form the legs of staples of the present disclosure to increase their rate of degradation. Suitable enzymes include, for example, peptide hydrolases such as elastase, cathepsin G, cathepsin E, cathepsin B, cathepsin H, cathepsin L, trypsin, pepsin, chymotrypsin, γ-glutamyltransferase (γ-GTP) and the like; sugar chain hydrolases such as phosphorylase, neuraminidase, dextranase, amylase, lysozyme, oligosaccharase and the like; oligonucleotide hydrolases such as alkaline phosphatase, endoribonuclease, endodeoxyribonuclease and the like. Where an enzyme is added, the enzyme may be included in a liposome or microsphere to control the rate of its release, thereby controlling the rate of degradation of the legs of a staple of the present disclosure. Methods for incorporating enzymes into liposomes and/or microspheres are within the purview of those skilled in the art.

Methods for closing a wound with staples of the present disclosure are also provided. A wound, such as an incision, may be closed with a row of staples of the present disclosure dispensed by any means within the purview of one skilled in the art, including conventional mechanical devices. The number of staples necessary to close a wound may vary depending upon.the configuration and length of the wound to be closed.

Staples of the present disclosure may possess certain advantages compared with skin staples known in the art. They require no significant modification of either insertion hardware or user procedures for insertion; the load bearing strength of the metallic central backspan portion is maintained; staple removal is required only for the central backspan portion; the pain-of removal is minimized since the bioabsorbable legs remain embedded; and the cost of staple removal can be eliminated since it can be carried out by the patient rather than by medical personnel.

While the above description contains many specifics, these specifics should not be construed as limitations on the scope of the disclosure, but merely as exemplifications of embodiments thereof. Those:skilled in the art will envision many other possibilities within the scope of the claims appended hereto.

## Claims

1. A method comprising:
providing a surgical staple comprising:
a backspan (4,6) comprising a non-absorbable material and two legs, at least a portion of each of the two legs comprising a bioabsorbable thermal shape memory polymeric material, wherein the said portions have a permanent bent or curved shape and are deformable to a temporary shape at a deforming temperature above the transition temperature (Ttrans) but below the permanent temperature (Tperm) of the thermal shape memory polymeric material; and
straightening the said portions to a temporary insertion shape at a deforming temperature above the transition temperature but below the permanent temperature of the thermal shape memory polymeric material.

2. The method of claim 1, wherein the shape memory polymer comprises a blend of materials selected from the group consisting of urethanes, lactic acid, glycolic acid, acrylates, caprolactones, homopolymers thereof, copolymers thereof, and combinations thereof.

3. The method of claim 1, wherein the bioabsorbable material is selected from the group consisting of caprolactones, dioxanones, diol esters, lactic acid, lactide, glycolic acid, glycolide, ether-ester diols, carbonates and combinations thereof.

4. The method of claim 1, wherein the bioabsorbable material is selected from the group consisting of urethanes blended with lactic acid and/or glycolic acid; acrylates blended with caprolactones and combinations thereof.

## Patentansprüche

1. Verfahren, dass umfasst:
Bereitstellen einer chirurgischen Klammer, die umfasst:
eine hintere Spanne (4, 6), die ein nicht-absorbierbares Material und zwei Schenkel umfasst, wobei wenigstens ein Abschnitt von jedem der beiden Schenkel ein bioabsorbierbares thermisches Formgedächtnis-Polymermaterial umfasst, wobei die Abschnitte eine dauerhaft gebogene oder gekrümmte Form aufweisen und bei einer Verformungstemperatur, die oberhalb der Umwandlungstemperatur (Tₜᵣₐₙₛ), jedoch unterhalb der Dauertemperatur (Tₚₑᵣₘ) des thermischen Formgedächtnis-Polymermaterials liegt, in eine temporäre Form verformt werden können, und
Geraderichten der Abschnitte in eine temporäre Einsetzform bei einer Verformungstemperatur, die oberhalb der Umwandlungstemperatur, jedoch unterhalb der Dauertemperatur des thermischen Formgedächtnis-Polymermaterials liegt.

2. Verfahren nach Anspruch 1, wobei das Formgedächtnis-Polymer eine Mischung von Materialien umfasst, die aus der Gruppe bestehend aus Urethanen, Milchsäure, Glycolsäure, Acrylaten, Caprolactonen, Homopolymeren davon, Copolymeren davon und Kombinationen davon ausgewählt ist.

3. Verfahren nach Anspruch 1, wobei das bioabsorbierbare Material aus der Gruppe bestehend aus Caprolactonen, Dioxanonen, Diolester, Milchsäure, Lactid, Glycolsäure, Glycolid, Ether-Ester Diolen, Carbonaten und Kombinationen davon ausgewählt ist.

4. Verfahren nach Anspruch 1, wobei das bioabsorbierbare Material aus der Gruppe bestehend aus Urethanen gemischt mit Milchsäure und/oder Glycolsäure, Acrylaten gemischt mit Caprolactonen und Kombinationen davon ausgewählt ist.

## Revendications

1. Procédé comprenant les étapes consistant à :
fournir une agrafe chirurgicale comprenant :
une portée arrière (4, 6) comprenant une matière non résorbable et deux pattes, au moins une partie de chacune des deux pattes comprenant un polymère à mémoire de forme thermique biorésorbable, lesdites parties étant dotées d'une forme pliée ou courbée permanente et pouvant être déformées suivant une forme temporaire à une température de déformation supérieure à la température de transition (Ttrans) mais inférieure à la température permanente (Tperm) du polymère à mémoire de forme thermique ; et
redresser lesdites parties suivant une forme temporaire d'insertion à une température de déformation supérieure à la température de transition mais inférieure à la température permanente du polymère à mémoire de forme thermique.

2. Procédé selon la revendication 1, dans lequel le polymère à mémoire de forme comprend un mélange de matières sélectionnées dans le groupe comprenant les uréthanes, l'acide lactique, l'acide glycolique, les acrylates, les caprolactones, leurs homopolymères, leurs copolymères et leurs combinaisons.

3. Procédé selon la revendication 1, dans lequel la matière biorésorbable est sélectionnée dans le groupe comprenant les caprolactones, les dioxanones, les esters de diol, l'acide lactique, le lactide, l'acide glycolique, le glycolide, les diols éther-ester, les carbonates et leurs combinaisons.

4. Procédé selon la revendication 1, dans lequel la matière biorésorbable est sélectionnée dans le groupe comprenant les uréthanes mélangés avec l'acide lactique et/ou l'acide glycolique ; les acrylates mélangés avec les caprolactones et leurs combinaisons.
